# EUROPEAN PATENT APPLICATION

(11) **EP 1 816 203 A1**
(43) Date of publication of application: **08.08.2007**
(21) Application number: 04822465.3
(22) Date of filing: 26.11.2004
(51) Int. Cl.: C12N 15/86, C12N 15/63, C12N 15/79, C12N 15/11, C12N 15/50, C12N 15/65, C12N 15/66, C12N 15/48, C12N 15/44

(54) **AN EXPRESSION VECTOR ENCODING CORONAVIRUS-LIKE PARTICLES**

(71) Applicant: DNA Shuttle Biopharm Co., Ltd., Taipei 106 (TW)
(72) Inventor: HWU, Luen, Taipei Taiwan 106 (TW)
(74) Representative: Svensson, Johan Henrik
(86) International application number: PCT/CN2004/001359
(87) International publication number: WO 2006/056103

(57) **Abstract**

The present invention provides an expression vector encoding coronavirus-like particles, which comprises two transcriptional units, wherein the first unit comprises genes encoding membrane M protein and envelope E protein from coronavirus, while the second unit comprises SpikeCT coding gene from coronavirus. Said expression vector is useful for cloning viral genes from Type I membrane fusion mechanism, and also is useful as DNA vaccine candidate.

## Description

### Field of the Invention

The invention relates to the field of recombinant DNA technology, more in particular to the field of DNA vaccine. In particular the invention relates to a new expression vector encoding a virus-like particle for cloning the Class I viral fusion protein gene and as DNA vaccine candidate.

### Background of the Invention

Vaccination has played a key role in the control of viral diseases during the past 30 years. Vaccination is based on a simple principle of immunity: once exposed to an infectious agent, an animal mounts an immune defense that protects against infection by the same agent. The goal of vaccination is to induce the animal to mount the defense prior to infection. Conventionally, this has been accomplished through the use of live attenuated or killed forms of the virus as immunogens. The success of these approaches in the past has been due in part to the presentation of native antigen and the ability of attenuated virus to elicit the complete range of immune responses obtained in natural infection. However, conventional vaccine methodologies have always been subject to a number of potential limitations. Attenuated strains can mutate to become more virulent or non-immunogenic; improperly inactivated vaccines may cause the disease that they are designed to prevent.

Recombinant DNA technology offers the potential for eliminating some of the limitations of conventional vaccines, by making possible the development of vaccines based on the use of defined antigens, rather than the intact infectious agent, as immunogens. These include peptide vaccines, consisting of chemically synthesized, immunoreactive epitopes; subunit vaccines, produced by expression of viral proteins in recombinant heterologous cells; and the use of live viral vectors for the presentation of one or a number of defined antigens. Both peptide and subunit vaccines are subject to a number of potential limitations. A major problem is the difficulty of ensuring that the conformation of the engineered proteins mimics that of the antigens in their natural environment. Suitable adjuvants and, in the case of peptides, carrier proteins, must be used to boost the immune response. In addition, these vaccines elicit primarily humoral responses, and thus may fail to evoke effective immunity.

Many different methods have been developed to introduce new genetic information into target cells. Currently, the most efficient means of introducing DNA into target cells is by employing modified viruses, so-called recombinant viral vectors. The most frequently used viral vector systems are based on retroviruses, adenoviruses, herpes viruses or the adeno-associated viruses (AAV). All systems have their specific advantages and disadvantages. Some of the vector systems possess the capacity to integrate their DNA into the host cell genome, whereas others do not. From some vector systems the viral genes can be completely removed from the vector while in other systems this is not yet possible. Some vector systems have very good in vivo delivery properties, while others do not. Some vector types are very easy to produce in large amounts, while others are very difficult to produce.

Coronaviruses carry three or four proteins in their envelopes. The M protein is the most abundant component. The small E protein is a minor but essential viral component. The importance of the S protein in pathogenesis is consistent with its biologic function in both viral entry and viral spread (Collins, A. R., et al., 1982, Virology 119:358-371; Williams, R. K., et al., 1991, Proc. Natl. Acad. Sci. USA 88:5533-5536). When expressed on the virion envelope, S protein binds to the cellular receptor and induces the fusion of viral and cell membranes during viral entry. Subsequent to infection, S protein expressed on the plasma membrane of infected cells induces cell-cell fusion. S protein also plays a role in the immune response to viral infection, as a target for neutralizing antibodies (Collins, A. R., et al., 1982, Virology 119:358-371) and as an inducer of a cell-mediated immunity (Bergmann, C. C., et al., 1996, J. Gen. Virol. 77:315-325; Castro, R. F., and S. Perlman, 1995, J. Virol. 69:8127-8131). The M and E proteins are the minimum protein units for virus assembly (Baudoux, P., et al., 1998, J. Virol. 72:8636-8643; Bos, E. C., 1996, Virology 218:52-60; de Haan, C. A. M., et al., 1998, J. Virol. 72:6838-6850; Godeke, G.-J., et al., 2000, J. Virol. 74:1566-1571; Vennema, H., et al., 1996, EMBO J. 15:2020-2028). Both are integral membrane proteins. The expression of M and E proteins together is sufficient to trigger the formation of virus-like particles (VLP). When S protein is coexpressed with M and E proteins, the S protein is incorporated into VLP with presumably authentic conformation. This has now been demonstrated for mouse hepatitis virus (MHV) (Bos, E. C., 1996, Virology 218:52-60; de Haan, C. A. M., et al., 1998, J. Virol. 72:6838-6850; Vennema, H., et al., 1996, EMBO J. 15:2020-2028), transmissible gastroenteritis virus (Baudoux, P., et al., 1998, J. Virol. 72:8636-8643), and feline infectious peritonitis virus (Godeke, G.-J., et al., 2000, J. Virol. 74:1566-1571). There is a hypothesis that the coronavirus membrane basically consists of a dense matrix of laterally interacting M proteins, which in some way requires the E protein for budding and in which the S and HE glycoproteins are incorporated, if available, by specific interactions with M via carboxyl terminal and the transmembrane region of Spike (de Haan, C. A. M., 1999, J. Virol. 73:7441-7452; Nguyen, V.-P., and B. G. Hogue. 1997, J. Virol. 71:9278-9284; Opstelten, D.-J. E., et al., 1995, J. Cell Biol. 131:339-349; Vennema, H., et al., 1996, EMBO J. 15:2020-2028). Such Spike-containing VLP can infect cells with the same infectivity like authentic virus (Bos, E. C., 1996, Virology 218:52-60). However, no effective DNA vectors are developed for use as DNA vaccine.

Therefore, there is a need to develop an effective DNA vector encoding virus-like particles that can present functional viral fusion protein in the surface of VLPs and these VLPs will be an excellent candidate as a potential vaccine against virus infection diseases.

### Summary Of the Invention

The present invention relates to an expression vector for cloning the Class I viral fusion protein gene and as DNA vaccine candidate, the expression vector comprising:
i) a first transcription unit comprising a membrane protein gene (M protein gene) of Coronavirus, an envelope protein gene (E protein gene) of Coronavirus and an internal ribosome entry sites (IRES) sequence, wherein the IRES is inserted into the junction of the membrane protein gene and the envelop protein gene;
ii) a first eukaryotic promoter operably linked to the membrane protein gene wherein the first promoter is located upstream of the M protein gene and drives the expression of the first transcription unit;
iii) a second transcription unit comprising a SpikeCT gene of Coronavirus and a multiple cloning site (MCS) for cloning or in-framed insertion of Class I viral fusion protein gene, wherein the MCS is located at the beginning of SpikeCT gene and has restriction enzymes cutting sites; and
iv) a second eukaryotic promoter operably linked to the SpikeCT gene wherein the second promoter is located upstream of the SpikeCT gene and drives the expression of the second transcription unit;
wherein the transcription activity of the first eukaryotic promoter is stronger than the second eukaryotic promoter.

### Brief Description of the Drawing

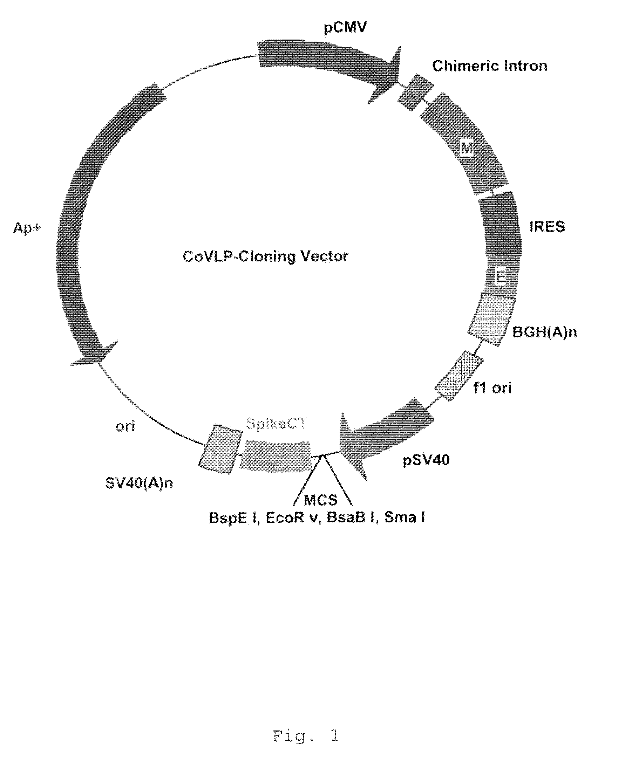

Figure 1 shows the plasmid map of one preferred embodiment of the expression vector of the invention.

### Detailed Description of the Invention

Genes encoding viral polypeptides capable of self assembly into defective, non-self propagating viral particles can be obtained from the genomic DNA of a DNA virus or the genomic cDNA of an RNA virus or from available subgenomic clones containing the genes. These genes will include those encoding viral capsid proteins (i.e., proteins that comprise the viral protein shell) and, in the case of enveloped viruses, such as retroviruses, the genes encoding viral envelope glycoproteins. The virus-like particles may be isolated and used themselves as immunogens for vaccination against pathogenic viruses, or for therapeutic purposes, such as enhancing immune responses in an infected individual, or for targeted delivery of therapeutic agents, such as cytotoxic drugs, to specific cell types.

The present invention provides an expression vector for cloning the Class I viral fusion protein gene and as DNA vaccine candidate, the expression vector comprising:
i) a first transcription unit comprising a membrane protein gene (M protein gene) of Coronavirus, an envelope protein gene (E protein gene) of Coronavirus and an internal ribosome entry sites (IRES) sequence, wherein the IRES is inserted into the junction of the membrane protein gene and the envelop protein gene;
ii) a first eukaryotic promoter operably linked to the membrane protein gene wherein the first promoter is located upstream of the M protein gene and drives the expression of the first transcription unit;
iii) a second transcription unit comprising a SpikeCT gene of Coronavirus and a multiple cloning site (MCS) for cloning or in-framed insertion of Class I viral fusion protein gene, wherein the MCS is located at the beginning of SpikeCT gene and has restriction enzymes cutting sites; and
iv) a second eukaryotic promoter operably linked to the SpikeCT gene wherein the second promoter is located upstream of the SpikeCT gene and drives the expression of the second transcription unit;
wherein the transcription activity of the first eukaryotic promoter is stronger than the second eukaryotic promoter.

According to the invention, the expression vector comprises a first transcription unit comprising a membrane protein gene (M protein gene) of Coronavirus, an envelope protein gene (E protein gene) of Coronavirus and an internal ribosome entry sites (IRES) sequence, wherein the IRES is inserted into the junction of the membrane protein gene and the envelop protein gene.

According to the invention, the envelope protein gene of the invention encodes envelope protein (E protein) of Coronavirus. The E protein is a small, envelope-associated protein. The E protein is a minor but essential viral component. In cells, it accumulates in and induces the coalescence of the membranes of the intermediate compartment (IC), giving rise to typical structures. A fraction of the proteins appear extracellularly in membranous structures of unknown identity. Preferably, the coronvirus of the invention is pig, human or avian coronavirus. More preferably, the coronavirus is pigTGEV coronavirus, human 229E coronavirus or human SARS virus. More preferably, the E protein gene of the invention has the sequence as set forth in SEQ ID N0:1.

The coding sequence of SARS E gene (SEQ ID NO:1)

ATGGCATACT CTTTTGTGTC TGAGGAAACT GGCACTCTGA TCGTGAACTC TGTACTGCTG
TTTCTCGCTT TTGTGGTATT CCTGCTGGTC ACTCTCGCTA TCCTCACTGC TCTTCGTCTG
TGTGCCTACT GTTGTAATAT CGTGAACGTG TCTCTGGTTA AGCCTACTGT GTATGTGTAT
TCTCGTGTGA AAAATCTCAA TTCTTCTGAA GGAGTTCCCG ATCTGCTGGT CTAG

According to the invention, the membrane protein gene of the invention encodes membrane protein (M protein) of Coronavirus. The M protein is the most abundant component; it is a type III glycoprotein consisting of a short amino-terminal ectodomain, three successive transmembrane domains, and a long carboxy-terminal domain on the inside of the virion (or in the cytoplasm). Preferably, the coronavirus of the invention is pig, human or avian coronavirus. More preferably, the coronavirus is pig TGEV coronavirus, human 229E coronavirus or human SARS virus. More preferably, the M protein gene of the invention has the sequence as set forth in SEQ ID N0:2.

The coding sequence of SARS M gene (SEQ ID NO:2)

ATGGCAGATA ACGGCACTAT TACTGTGGAG GAACTGAAAC AACTGCTGGA ACAATGGAAC
CTCGTAATCG GCTTTCTCTT TCTGGCTTGG ATTATGTTGT TACAGTTTGC GTATTCTAAT
CGTAACCGTT TCCTCTACAT TATTAAGCTC GTTTTCCTGT GGTTGTTGTG GCCTGTAACT
CTTGCTTGCT TTGTGCTTGC TGCTGTCTAT CGTATCAACT GGGTTACTGG TGGTATTGCT
ATCGCTATGG CTTGTATTGT AGGCTTGATG TGGCTGTCTT ATTTCGTTGC TTCTTTCCGT
CTGTTTGCTC GTACTCGCTC TATGTGGTCC TTTAATCCTG AGACTAATAT CCTGCTGAAT
GTTCCGCTCC GTGGTACTAT CGTTACTAGA CCGCTGATGG AATCTGAACT GGTTATTGGT
GCCGTCATTA TCCGTGGTCA TTTGCGTATG GCTGGTCACT CTCTGGGTCG TTGCGATATT
AAGGATCTGC CAAAGGAAAT CACTGTAGCC ACTTCTCGTA CTCTGTCTTA CTATAAACTC
GGTGCATCGC AACGTGTGGG AACTGATTCG GGCTTCGCTG CGTATAATCG TTATCGTATT
GGCAACTATA AACTGAACAC CGACCACGCA GGCTCTAATG ACAACATCGC TCTCCTCGTT
CAGTGA

According to the invention, for assembly of the coronavirus envelope, only the M protein and the E protein are needed (Cornelis A. M. de Haan et al., Journal of Virology, June 2000, p. 4967-4978). Expression in cells of the genes coding for these proteins leads to the formation and release of viruslike particles (VLPs) similar in size and shape to authentic virions.

According to the invention, the first transcription unit comprises an internal ribosome entry sites (IRES) sequence that is inserted into the junction of M gene and E gene. The IRES allows the translation of two or more proteins from a di- or polycistronic mRNA. The IRES units are fused to 5' ends of one or more additional coding sequences which are then inserted into the vectors at the end of the original coding sequence, so that the coding sequences are separated from one another by IRES. According to the invention, any IRES derivatives also can be used in the plasmid construct of the invention. The IRES sequence allows the ribosomal machinery to initiate translation from a secondary site, within a single transcript.

According to the invention, the expression vector of the invention comprises a first eukaryotic promoter operably linked to the membrane protein gene wherein the first promoter is located upstream of the M protein gene and drives the expression of the first transcription unit. Preferably, the first eukaryotic promoter is viral derived promoter like CMV, SV40, RSV, HIV-1 LTR and the hybrid Beta-actin/CMV promoter enhancer and muscle-specific desmin, creatine kinase, beta-Actin promoter and other ubiquitous expression promoter like EF1 alpha, Ubiquitin promoter. The most preferably, the first eukaryotic promoter is pCMV, Hybrid Beta-actin/CMV promoter enhancer, beta-Actin promoter.

According to the invention, a second transcription unit comprises a SpikeCT gene and a multiple cloning site (MCS) having restriction enzymes cutting sites, wherein the MCS is located at the beginning of SpikeCT gene. The SpikeCT gene interacts with the M protein interaction domain, which encodes C-terminal heptad repeat located upstream of an aromatic residue-rich region juxtaposed to the transmembrane segment of Spike protein of coronavirus. The multiple cloning site (MCS) has several restriction enzymes cutting sites. The restriction enzyme cutting site includes, but not limited to, Smal, BsaB I, EcoR V and BspE I. The MCS is located at the beginning of SpikeCT gene and can be used for cloning or in-framed insertion of Class I viral fusion protein gene. The Class I viral fusion protein gene includes, but not limited to, gp160 of HIV, HA of influenza virus and Spike of SARS virus. The Class I viral fusion protein gene is derived form the viruses that adapt Class I viral fusion mechanism comprises coronavirus, HIV, and influenza virus, into cloning /expression vector of the invention and then used as DNA vaccine candidate. Preferably, the SpikeCT gene having the sequence as set forth in SED ID No:3.

The coding sequence of SpikeCT gene (SEQ ID NO:3)

GATATCTCCGGA ATCAATGCGA GCGTAGTGAA CATCCAGAAA
GAGATTGACC GTTTGAATGA AGTTGCTAAA AATCTGAATG AACCTCTGAT TGACCTCCAA
GAACTCGGCA AATATGAGCA ATACATTAAA TGGCCTTGGT ATGTCTGGTT GGGTTTCATC

GCAGGTCTCA TCGCTATCGT TATGGTGACT ATTCTGCTGT GTTGTATGAC TTCTTGTTGC
TCTTGTCTGA AAGGTGCGTG TTCTTGTGGT TCTTGTTGTA AGTTTGATGA GGATGATTCT
GAGCCAGTTC TGAAGGGTGT GAAGCTGCAT TATACCTAGT TCGAA

According to the invention, the expression vector of the invention can produce a virus-like particle and express the functional fusion gene of the Class I viruses on the surface of virus-like particle. Thus, there are several restriction enzymes cutting sites (multiple cloning site ,MCS) including, but not limiting to, Smal, BsaB I, EcoR V, BspE I located at the beginning of "SpikeCT" gene and these sites can be used for insertion or cloning Class I viral fusion protein gene into the cloning /expression vector of the invention and then used as DNA vaccine candidate. The viruses that adapt Class I viral fusion mechanism includes, but not limited to, coronavirus, HIV, influenza virus, and so on.

According to the invention, the second promoter is located upstream of the SpikeCT gene and drives the expression of the second transcription unit. Preferably, the second eukaryotic promoter is viral derived promoter like CMV, SV40, RSV, HIV-1 LTR and the hybrid Beta-actin/CMV promoter enhancer and muscle-specific desmin, creatine kinase, beta-Actin promoter and other ubiquitous orcontitutive expression promoter like EF1alpha, Ubiquitin promoter. The most preferably, the second eukaryotic promoter is pCMV, SV40, beta-Actin and EF1alpha promoter.

According to the invention, the transcription units of the invention comprise a polyA signal. Persons skilled in the art can recognize that any transcription has the polyA signal. Preferably, the transcription units of the invention comprises BGH polyA signal.

According to the invention, the expression vector further comprises a replication origin for the propagation plasmid in a host cell. The determination of the replication origin depends on the types of the host cells.

According to the invention, the expression vector of the invention further comprises an antibiotics-resistant gene as the selection marker.

According to the invention, the expression vector of the invention can spontaneously form virus-like particles. The virus-like particles can be used as vaccine candidate against virus infection. The virus-like particle lacks the genetic materials of the virus. Therefore, such particles do not have infectious ability. The expression vector of the invention can be administrated to animals and transfected with host cells to produce the virus-like particles.

Most viruses consist of few proteins only with structural restrictions impinged on them. In fact, using these few proteins, viruses are forced to generate a quasi-crystalline, highly repetitive surface. Such highly repetitive antigens efficiently cross-link B cell receptors, a process which generates strong activation signal in B cells. In contrast, self-antigens are usually not highly organized, in particular not those accessible to B cells. Thus, B cells use antigen organization as a marker for infectious non-self. Vaccines based on virus like particles (VLPs) exploit this basic phenomenon, since VLPs exhibit surfaces as repetitive and organized as those of viruses. Consequently, similar to viruses, VLPs are able to trigger strong B cell responses in the absence of adjuvants. Based on the above principle, the virus-like particles produced through the expression of the expression vector of the invention in the cells can be used as a vaccine candidate against virus diseases.

### EXAMPLE

### E gene synthesized by PCR

The polymerase chain reaction (PCR) was used to synthesize the E gene of SARS virus. The PCR template(0.1 pmole) of SARS E gene is the mixture of primers that listed below and PCR reaction was performed using the standard PCR method described by Innis et al. (PCR protocols. A guide to methods and applications, 1990, Academic Press) using KOD Taq polymerase (Novagene.com). The PCR primers are shown below:
5'-ACC ATG GCA TAC TCT TTT GTG TCT GAG GAA ACT G-3'* (SEQ ID NO: 4)
   E1L 
5'-ACA GCA GTA CAG AGT TCA CGA TCA GAG TGC CAG TTT CCT CAG
ACA CAA AAG-3' (SEQ ID NO: 5)
   E2L 
5'-TGA CCA GCA GGA ATA CCA CAA AAG CGA GAA ACA GCA GTA CAG
AGT TCA CGA-3' (SEQ ID NO: 6)
   E3L 
5'-GCA CAC AGA CGA AGA GCA GTG AGG ATA GCG AGA GTG ACC AGC
AGG AAT ACC ACA-3' (SEQ ID NO: 7)
   E4L 
5'-ACC AGA GAC ACG TTC ACG ATA TTA CAA CAG TAG GCA CAC AGA
CGA AGA GCA G-3' (SEQ ID NO: 8)
   E5L 
5'-GAT TTT TCA CAC GAG AAT ACA CAT ACA CAG TAG GCT TAA CCA
GAG ACA CGT TCA CGA T-3'* (SEQ ID NO: 9)
   E6L 
5'-TCT AGA CCA GCA GAT CGG GAA CTC CTT CAG AAG AAT TGA GAT
TTT TCA CAC GAG AAT ACA CA-3' (SEQ ID NO: 10)
   E7L
      5'-TCT AGA CCA GCA GAT CGG GA-3' (SEQ ID NO: 11)

The DNA template is initial denaturized at: 95° C for 3 minutes. The PCR conditions are listed as follow:

### First PCR reaction with 10cycles of 3 steps

1.Annealing: 58°C for 20 seconds
2. Extension: 72° C for 40 seconds
3.Denaturation: 95°C for 1 minutes

### Second PCR reaction with 20 cycles of 3 steps

1.Denaturation: 95°C for 1 minutes
2.Annealing: 62° C for 20 seconds
3.Extension: 72 ° C for 40 seconds

The resultant double-stranded full-length of E gene products were analyzed on 1.2% agarose gel and purified with QlAquick PCR purification kit (Qiagen Inc.) and then ligated with pGEM-T vector (Promega Co.) to obtain pGEM(T)/E^{A+} clones. The sequence of E gene is as set forth in SEQ ID NO: 1.

### M gene synthesized by splicing-over PCR

The synthesis of M gene of SARS virus was similar to the PCR method as mentioned in the above and the PCR template (0.1 pmole) of SARS M gene is the mixture of primers that listed below by using KOD Taq polymerase (Novagene.com) are as follows:
The PCR primers are listed as follows:
M1-1U
   5'-TGA TCA TGG CAG ATA ACG GCA C-3' (SEQ ID NO: 12)
M1U 
5'-TGA TCA TGG CAG ATA ACG GCA CTA TTA CTG TGG
AGG A-3' (SEQ ID NO: 13)
M1L 
5'-GTT CCA TTG TTC CAG CAG TTG TTT CAG TTC CTC
CAC AGT AAT AGT GCC G-3' (SEQ ID NO: 14)
M2L 
5'-AAG CCA GAA AGA GAA AGC CGA TTA CGA GGT TCC
ATT GTT CCA GCA GTT G-3' (SEQ ID NO: 15)
M3L 
5'-AGA ATA CGC AAA CTG TAA CAA CAT AAT CCA AGC
CAG AAA GAG AAA GCC GA-3' (SEQ ID NO: 16)
M4L 
5'-CGA GCT TAA TAA TGT AGA GGA AAC GGT TAC GAT
TAG AAT ACG CAA ACT GTA ACA ACA-3' (SEQ ID NO: 17)
M5L 
5'-CAA GAG TTA CAG GCC ACA ACA ACC ACA GGA AAA
CGA GCT TAA TAA TGT AGA GGA AA-3' (SEQ ID NO: 18)
M6L 
5'-TTG ATA CGA TAG ACA GCA GCA AGC ACA AAG CAA
GCA AGA GTT ACA GGC CAC AAC A-3' (SEQ ID NO: 19)
M7L 
5'-CAA GCC ATA GCG ATA GCA ATA CCA CCA GTA ACC
CAG TTG ATA CGA TAG ACA GCA GCA A-3' (SEQ ID NO: 20)
M8L 
5'-AAC GAA ATA AGA CAG CCA CAT CAA GCC TAC AAT
ACA AGC CAT AGC GAT AGC AAT AC-3' (SEQ ID NO: 21)
M9L 
5'-ACA TAG AGC GAG TAC GAG CAA ACA GAC GGA AAG
AAG CAA CGA AAT AAG ACA GCC ACA TC-3' (SEQ ID NO: 22)
M10U
   5'-TCC TGC TGA ATG TTC CGC TC-3' (SEQ ID NO: 23)
M10L 
5'-GAG CGG AAC ATT CAG CAG GAT ATT AGT CTC AGG
ATT AAA GGA CCA CAT AGA GCG AGT ACG AGC AA-3' (SEQ ID NO: 24)
M11L 
5'-CAT CAG CGG TCT AGT AAC GAT AGT ACC ACG GAG
CGG AAC ATT CAG CAG GA-3' (SEQ ID NO: 25)
M12L 
5'-ACC ACG GAT AAT GAC GGC ACC AAT AAC CAG TTC
AGA TTC CAT CAG CGG TCT AGT AAC GAT-3' (SEQ ID NO: 26)
M13L
   5'-ATA CGC AAA TGA CCA CGG ATA ATG ACG GCA C-3' (SEQ ID NO: 27) M14L 
5'-AAC GAC CCA GAG AGT GAC CAG CCA TAC GCA AAT
GAC CAC GGA TAA-3' (SEQ ID NO: 28)
M15L 
5'-TAC AGT GAT TTC CTT TGG CAG ATC CTT AAT ATC
GCA ACG ACC CAG AGA GTG-3' (SEQ ID NO: 29)
M16L 
5'-CCG AGT TTA TAG TAA GAC AGA GTA CGA GAA GTG
GCT ACA GTG ATT TCC TTT GGC AGA-3' (SEQ ID NO: 30)
M17L 
5'-CGA AGC CCG AAT CAG TTC CCA CAC GTT GCG ATG
CAC CGA GTT TAT AGT AAG ACA GAG T-3' (SEQ ID NO: 31)
M18L 
5'-CAG TTT ATA GTT GCC AAT ACG ATA ACG ATT ATA
CGC AGC GAA GCC CGA ATC AGT TCC C-3' (SEQ ID NO: 32)
 
M19L5'-GAG AGC GAT GTT GTC ATT AGA GCC TGC GTG GTC
GGT GTT CAG TTT ATA GTT GCC AAT ACG AT-3' (SEQ ID NO: 33)
M20L

5'-TCA CTG AAC GAG GAG AGC GAT GTT GTC ATT AGA
G-3' (SEQ ID NO: 34)

The PCR conditions are essentially as mentioned above. The resultant double-stranded full-length of M gene products were analyzed on 1.0% agarose gel and purified with QlAquick PCR purification or gel extraction kit (Qiagen Inc.) and then ligated with pGEM-T vector (Promega Co.) to obtain pGEM(T)/M clones. The sequence of M gene is as set forth in SEQ ID NO: 2.

The "SpikeCT" gene Encoding a heptad region 2 and transmembrane domain of Spike Protein Gene of SARS Virus Synthesized by PCR.

The PCR method and condition, purification and cloning of the PCR products are as mentioned above. The DNA template (0.1 pmole) of SpikeCT gene is the mixture of primers listed below:
The PCR primers are listed as follows:
   SpikeCTup-EcoRV
   5'-GAT ATC TCC GGA ATC AAT GCG AGC GT-3' (SEQ ID NO: 35)
   DI-1 U/BspE I 
5'-5'-TCC GGA ATC AAT GCG AGC GTA GTG AAC ATC CAG
AA-3' (SEQ ID NO: 36)
   DI-1 L 
5'-GCA ACT TCA TTC AAA CGG TCA ATC TCT TTC TGG ATG TTC
ACT ACG CTC-3' (SEQ ID NO: 37)
   DI-2L 
5'-ATC AGA GAT TCA TTC AGA TTT TTA GCA ACT TCA TTC AAA
CGG TCA-3' (SEQ ID NO: 38)
   DI-3L 
5'-TCA TAT TTG CCG AGT TCT TGG AGG TCA ATC AGA GAT TCA
TTC AGA TTT TTA-3' (SEQ ID NO: 39)
   DI-4L 
5'-CAA GGC CAT TTA ATG TAT TGC TCA TAT TTG CCG AGT TCT
TGG A-3' (SEQ ID NO: 40)
   DI-5L 
5'-ACC TGC GAT GAA ACC CAA CCA GAC ATA CCA AGG CCA TTT AAT GTA
TTG CT-3' (SEQ ID NO: 41)
   DI-6L 
5'-CAG AAT AGT CAC CAT AAC GAT AGC GAT GAG ACC TGC GAT GAA ACC
CAA CC-3' (SEQ ID NO: 42)
   DI-7L 
5'-AGA GCA ACA AGA AGT CAT ACA ACA CAG CAG AAT AGT CAC CAT AAC
GAT AG-3' (SEQ ID NO: 43)
   DI-8L 
5'-ACC ACA AGA ACA CGC ACC TTT CAG ACA AGA GCA ACA AGA AGT CAT
ACA AC-3' (SEQ ID NO: 44)
   DI-9L 
5'-GAA TCA TCC TCA TCA AAC TTA CAA CAA GAA CCA CAA GAA CAC GCA
CCT TT-3' (SEQ ID NO: 45)
   DI-10L 
5'-GCT TCA CAC CCT TCA GAA CTG GCT CAG AAT CAT CCT CAT
CAA ACT TAC A-3' (SEQ ID NO: 46)
   DI-11L
      5'-CCT AGG TAT AAT GCA GCT TCA CAC CCT TCA GAA CT-3' (SEQ ID NO: 47) SpikeCTdn-BstBI
   5'- TTC GAACT AGG TAT AAT GCA GCT TCA C -3' (SEQ ID NO: 48)

The PCR conditions are as mentioned above. The resultant double-stranded SCT320 gene products were purified with Qiagen/PCR purification kit (Qiagen Inc.) and cloned with pGEM-T vector (Promega Co.) to obtain pGEM(T)/SpikeCT(EcoRV/BstBI). The pGEM(T)/ SpikeCT (EcoRV/BstBI) plasmid in which containing a gene encodes C-terminal heptad repeat located upstream of an aromatic residue-rich region juxtaposed to the transmembrane segment of Spike protein of SARS coronavirus. The coding sequence of "SCT320" gene is as set forth in SEQ ID NO: 3.

### Construction of Expression vector of the Invention

The manipulation of DNA is instructed by Sambrook and Russell et al (Molecular cloning third edition), Cold Spring Harbor Laboratory Express. The restriction enzymes, T4DNA ligase, Klenow enzyme are purchased from New England Biolab.com and performed by manufacture's recommendation.

### I. Construction of pCMV promoter-driven SARS M gene plasmid

The pGEM(T)/M clone was used as DNA template for PCR with T7 and SP6 promoter primer, and the PCR condition, except the Annealing temperature was set at 50□ for 20 seconds, are same as mentioned above. The resulting M gene PCR products were purified with QlAquick PCR purification kit (Qiagen Inc.) and digested with Bcl I and Notl to obtain DNA inserts containing SARS M gene. The pEGFP-N1 plasmid (Clontech Co.) was digested with BgIII and Not I and the resulting Bgl II , Not I digested pEGFP-N1 plasmid were uesd as DNA vector for ligation with Bcl I , Not I digested SARS M gene DNA inserts to obtain pN1/M DNA plasmid. The pN1/M DNA plasmid was digested with Nhel and Not I to obtain DNA inserts containing SARS M gene. The pACT plasmid (Promega Co.) was digested with Nhe I and Not I. The resulting Nhe I, Not I digested pACT DNA vector were ligated with Nhe I, Not I digested SARS M gene DNA inserts to obtain pACT/M DNA plasmid. The pACT/M DNA plasmid was digested with Bgl II and Asp 718 to obtain DNA inserts containing CMV promoter and SARS M gene. The pCDNA3.1 (+) plasmid (Invitrogen Co.) was digested with BgIII and Asp718. The resulting BgIII, Asp718 digested pCDNA3.1 (+)DNA vector were ligated with BgIII, Asp718 digested DNA containing CMV promoter and SARS M gene inserts to obtain pCDNA 3.1+M DNA plasmid.

### II. Constructing IRES-SARS E gene plasmid

The pIRES2-EGFP (Clontech Co.) was digested with Xho I and Nco I to get "IRES" DNA insert. The GL3basic vector (Promega Co.) was digested with Xho I and Nco I. The Xho I,Nco I digested pGL3basic DNA vector were ligated with Xho I,Nco I digested IRES DNA insert by using T4 DNA ligase (NEB Co.). The ligation mix was transformed into *E. coli* DH5αcompetent cells to obtain pGL3B/IRES-luciferase DNA plasmid. The T/E^{A+} clone, containing SARS E gene with second Alanine insertion mutant, was digested with Ncol and Xbal to obtain SARS E gene DNA inserts. The pGL3B/IRES-luciferase DNA plasmid was digested with Ncol and Xbal. The Nco I, Xba I digested pGL3B/IRES-luciferase DNA vector was ligated with Nco I, Xba I digested SARS E^{A+} gene DNA inserts to get pGL3B/IRES-E^{A+} DNA plasmid.

### III. Construction of pCMV-driven M+E gene expression plasmid (CoronaVirus-Like Particle (CoVLP) expression vector)

The pGL3B/IRES-E^{A+} DNA plasmid was digested with BamHl and Xbal to obtain DNA inserts containing IRES and SARS E^{A+} gene. The pCDNA3.1+M DNA plasmid was digested with BamHl and Xbal. The resultant BamH I, Xba I digested pCDNA3.1+M DNA vector were ligated with BamH I, Xba I digested DNA inserts containing IRES and SARS E^{A+} fused gene to obtain pCDNA3.1+M/IRES-E^{A+} plasmid.

### IV. Constructing the DNA vector of invention

The pGEM(T)/ SpikeCT (EcoRV/BstBI) plasmid, in which containing a gene encodes C-terminal heptad repeat located upstream of an aromatic residue-rich region juxtaposed to the transmembrane segment of Spike protein of SARS coronavirus, was digested with Spe I and filled-in by Klenow enzyme and dNTPs. After separated onto 1.2% agarose gel, the DNA fragment were purified with QlAquick gel extraction kit (QIAGEN. Com.) and further digested with BstB I. The resultant "SpikeCT" gene fragments were used as DNA inserts and ligated with BsaB I, BstB I digested, gel purified pCDNA3.1+M/IRES-E^{A+} DNA vector plasmid. After transformation into E. coli DH5alpha cells, get the DNA vector of invention, so-called CoVLP-cloning vector. There are several restriction enzymes cutting sites (multiple cloning site, MCS) including Smal, BsaB I, EcoR V, BspE I located at the beginning of "SpikeCT" gene and these sites can be used for cloning or in-frame fused Class I viral fusion protein gene with SpikeCT gene in the vector of invention and then the resultant DNA plasmid can be used as DNA vaccine candidate. The map of the CoVLP-cloning vector is shown in Figure 1. The sequence of the CoVLP-cloning vector is as set forth in SEQ ID N0:49.

The DNA sequence of CoVLP cloning vector (pCDNA3.1+M+IRES-E+SpikeCT) (SEQ ID NO:49)

GACGGATCGGGAGATCTTCAATATTGGCCATTAGCCATATTATTCATTGGTTATATAGCATA
AATCAATATTGGCTATTG

GCCATTGCATACGTTGTATCTATATCATAATATGTACATTTATATTGGCTCATGTCCAATATG
ACCGCCATGTTGGCATT

GATTATTGACTAGTTATTAATAGTAATCAATTACGGGGTCATTAGTTCATAGCCCATATATGG
AGTTCCGCGTTACATAA

CTTACGGTAAATGGCCCGCCTGGCTGACCGCCCAACGACCCCCGCCCATTGACGTCAATA
ATGACGTATGTTCCCATAGT

AACGCCAATAGGGACTTTCCATTGACGTCAATGGGTGGAGTATTTACGGTAAACTGCCCAC
TTGGCAGTACATCAAGTGT

ATCATATGCCAAGTCCGCCCCCTATTGACGTCAATGACGGTAAATGGCCCGCCTGGCATTA
TGCCCAGTACATGACCTTA

CGGGACTTTCCTACTTGGCAGTACATCTACGTATTAGTCATCGCTATTACCATGGTGATGCG
GTTTTGGCAGTACACCAA

TGGGCGTGGATAGCGGTTTGACTCACGGGGATTTCCAAGTCTCCACCCCATTGACGTCAAT
GGGAGTTTGTTTTGGCACC

AAAATCAACGGGACTTTCCAAAATGTCGTAACAACTGCGATCGCCCGCCCCGTTGACGCAA
ATGGGCGGTAGGCGTGTAC

GGTGGGAGGTCTATATAAGCAGAGCTCGTTTAGTGAACCGTCAGATCACTAGAAGCTTTAT
TGCGGTAGTTTATCACAGT

TAAATTGCTAACGCAGTCAGTGCTTCTGACACAACAGTCTCGAACTTAAGCTGCAGTGACTC
TCTTAAGGTAGCCTTGCA

GAAGTTGGTCGTGAGGCACTGGGCAGGTAAGTATCAAGGTTACAAGACAGGTTTAAGGAG
ACCAATAGAAACTGGGCTTG

TCGAGACAGAGAAGACTCTTGCGTTTCTGATAGGCACCTATTGGTCTTACTGACATCCACTT
TGCCTTTCTCTCCACAGG

TGTCCACTCCCAGTTCAATTACAGCTCTTAAGGCTAGAGTACTTAATACGACTCACTATAGG
CTAGCGCTACCGGACTCA

GATCATGGCAGATAACGGCACTATTACTGTGGAGGAACTGAAACAACTGCTGGAACAATGG
AACCTCGTAATCGGCTTTC

TCTTTCTGGCTTGGATTATGTTGTTACAGTTTGCGTATTCTAATCGTAACCGTTTCCTCTACA
TTATTAAGCTCGTTTTC

CTGTGGTTGTTGTGGCCTGTAACTCTTGCTTGCTTTGTGCTTGCTGCTGTCTATCGTATCAA
CTGGGTTACTGGTGGTAT

TGCTATCGCTATGGCTTGTATTGTAGGCTTGATGTGGCTGTCTTATTTCGTTGCTTCTTTCC
GTCTGTTTGCTCGTACTC

GCTCTATGTGGTCCTTTAATCCTGAGACTAATATCCTGCTGAATGTTCCGCTCCGTGGTACT
ATCGTTACTAGACCGCTG

ATGGAATCTGAACTGGTTATTGGTGCCGTCATTATCCGTGGTCATTTGCGTATGGCTGGTCA
CTCTCTGGGTCGTTGCGA

TATTAAGGATCTGCCAAAGGAAATCACTGTAGCCACTTCTCGTACTCTGTCTTACTATAAACT
CGGTGCATCGCAACGTG

TGGGAACTGATTCGGGCTTCGCTGCGTATAATCGTTATCGTATTGGCAACTATAAACTGAAC
ACCGACCACGCAGGCTCT

AATGACAACATCGCTCTCCTCGTTCAGTGAAATCACTAGTGCGGCCGCAGGTACCGAGCTC
GGATCCGCCCCTCTCCCTC

CCCCCCCCCTAACGTTACTGGCCGAAGCCGCTTGGAATAAGGCCGGTGTGCGTTTGTCTAT
ATGTTATTTTCCACCATAT

TGCCGTCTTTTGGCAATGTGAGGGCCCGGAAACCTGGCCCTGTCTTCTTGACGAGCATTCC
TAGGGGTCTTTCCCCTCTC

GCCAAAGGAATGCAAGGTCTGTTGAATGTCGTGAAGGAAGCAGTTCCTCTGGAAGCTTCTT
GAAGACAAACAACGTCTGT

AGCGACCCTTTGCAGGCAGCGGAACCCCCCACCTGGCGACAGGTGCCTCTGCGGCCAAAA
GCCACGTGTATAAGATACAC

CTGCAAAGGCGGCACAACCCCAGTGCCACGTTGTGAGTTGGATAGTTGTGGAAAGAGTCA
AATGGCTCTCCTCAAGCGTA

TTCAACAAGGGGCTGAAGGATGCCCAGAAGGTACCCCATTGTATGGGATCTGATCTGGGG
CCTCGGTGCACATGCTTTAC

ATGTGTTTAGTCGAGGTTAAAAAAACGTCTAGGCCCCCGAACCACGGGGACGTGGTTTTC
CTTTGAAAAACACGATGAT

AATATGGCCACAACCATGGCATACTCTTTTGTGTCTGAGGAAACTGGCACTCTGATCGTGAA
CTCTGTACTGCTGTTTCT

CGCTTTTGTGGTATTCCTGCTGGTCACTCTCGCTATCCTCACTGCTCTTCGTCTGTGTGCCT
ACTGTTGTAATATCGTGA

ACGTGTCTCTGGTTAAGCCTACTGTGTATGTGTATTCTCGTGTGAAAAATCTCAATTCTTCTG
AAGGAGTTCCCGATCTG

CTGGTCTAGAGGGCCCGTTTAAACCCGCTGATCAGCCTCGACTGTGCCTTCTAGTTGCCAG
CCATCTGTTGTTTGCCCCT

CCCCCGTGCCTTCCTTGACCCTGGAAGGTGCCACTCCCACTGTCCTTTCCTAATAAAATGA
GGAAATTGCATCGCATTGT

CTGAGTAGGTGTCATTCTATTCTGGGGGGTGGGGTGGGGCAGGACAGCAAGGGGGAGGA
TTGGGAAGACAATAGCAGGCA

TGCTGGGGATGCGGTGGGCTCTATGGCTTCTGAGGCGGAAAGAACCAGCTGGGGCTCTAG
GGGGTATCCCCACGCGCCCT

GTAGCGGCGCATTAAGCGCGGCGGGTGTGGTGGTTACGCGCAGCGTGACCGCTACACTT
GCCAGCGCCCTAGCGCCCGCT

CCTTTCGCTTTCTTCCCTTCCTTTCTCGCCACGTTCGCCGGCTTTCCCCGTCAAGCTCTAAA
TCGGGGGCTCCCTTTAGG

GTTCCGATTTAGTGCTTTACGGCACCTCGACCCCAAAAAACTTGATTAGGGTGATGGTTCAC
GTAGTGGGCCATCGCCCT

GATAGACGGTTTTTCGCCCTTTGACGTTGGAGTCCACGTTCTTTAATAGTGGACTCTTGTTC
CAAACTGGAACAACACTC

AACCCTATCTCGGTCTATTCTTTTGATTTATAAGGGATTTTGCCGATTTCGGCCTATTGGTTA
AAAAATGAGCTGATTTA

ACAAAAATTTAACGCGAATTAATTCTGTGGAATGTGTGTCAGTTAGGGTGTGGAAAGTCCCC
AGGCTCCCCAGCAGGCAG

AAGTATGCAAAGCATGCATCTCAATTAGTCAGCAACCAGGTGTGGAAAGTCCCCAGGCTCC
CCAGCAGGCAGAAGTATGC

AAAGCATGCATCTCAATTAGTCAGCAACCATAGTCCCGCCCCTAACTCCGCCCATCCCGCC
CCTAACTCCGCCCAGTTCC

GCCCATTCTCCGCCCCATGGCTGACTAATTTTTTTTATTTATGCAGAGGCCGAGGCCGCCT
CTGCCTCTGAGCTATTCCA

GAAGTAGTGAGGAGGCTTTTTTGGAGGCCTAGGCTTTTGCAAAAAGCTCCCGGGAGCTTGT
ATATCCATTTTCGGATCTG

ATCAAGAGACAGGATGACTAGTGATTGATATCTCCGGAATCAATGCGAGCGTAGTGAACAT
CCAGAAAGAGATTGACCGT

TTGAATGAAGTTGCTAAAAATCTGAATGAACCTCTGATTGACCTCCAAGAACTCGGCAAATA
TGAGCAATACATTAAATG

GCCTTGGTATGTCTGGTTGGGTTTCATCGCAGGTCTCATCGCTATCGTTATGGTGACTATTC
TGCTGTGTTGTATGACTT

CTTGTTGCTCTTGTCTGAAAGGTGCGTGTTCTTGTGGTTCTTGTTGTAAGTTTGATGAGGAT
GATTCTGAGCCAGTTCTG

AAGGGTGTGAAGCTGCATTATACCTAGttcgaaATGACCGACCAAGCGACGCCCAACCTGCCA
TCACGAGATTTCGATTC

CACCGCCGCCTTCTATGAAAGGTTGGGCTTCGGAATCGTTTTCCGGGACGCCGGCTGGAT
GATCCTCCAGCGCGGGGATC

TCATGCTGGAGTTCTTCGCCCACCCCAACTTGTTTATTGCAGCTTATAATGGTTACAAATAAA
GCAATAGCATCACAAAT

TTCACAAATAAAGCATTTTTTTCACTGCATTCTAGTTGTGGTTTGTCCAAACTCATCAATGTAT
CTTATCATGTCTGTAT

ACCGTCGACCTCTAGCTAGAGCTTGGCGTAATCATGGTCATAGCTGTTTCCTGTGTGAAATT
GTTATCCGCTCACAATTC

CACACAACATACGAGCCGGAAGCATAAAGTGTAAAGCCTGGGGTGCCTAATGAGTGAGCTA
ACTCACATTAATTGCGTTG

CGCTCACTGCCCGCTTTCCAGTCGGGAAACCTGTCGTGCCAGCTGCATTAATGAATCGGCC
AACGCGCGGGGAGAGGCGG

TTTGCGTATTGGGCGCTCTTCCGCTTCCTCGCTCACTGACTCGCTGCGCTCGGTCGTTCGG
CTGCGGCGAGCGGTATCAG

CTCACTCAAAGGCGGTAATACGGTTATCCACAGAATCAGGGGATAACGCAGGAAAGAACAT
GTGAGCAAAAGGCCAGCAA

## Claims

1. An expression vector for cloning the Class I viral fusion protein gene and as DNA vaccine candidate, the expression vector comprising:
i) a first transcription unit comprising a membrane protein gene (M protein gene) of Coronavirus, an envelope protein gene (E protein gene) of Coronavirus and an internal ribosome entry sites (IRES) sequence, wherein the IRES is inserted into the junction of the membrane protein gene and the envelop protein gene;
ii) a first eukaryotic promoter operably linked to the membrane protein gene wherein the first promoter is located upstream of the M protein gene and drives the expression of the first transcription unit;
iii) a second transcription unit comprising a SpikeCT gene of Coronavirus and a multiple cloning site (MCS) for cloning or in-framed insertion of Class I viral fusion protein gene, wherein the MCS is located at the beginning of SpikeCT gene and has restriction enzymes cutting sites; and
iv) a second eukaryotic promoter operably linked to the SpikeCT gene wherein the second promoter is located upstream of the SpikeCT gene and drives the expression of the second transcription unit;
wherein the transcription activity of the first eukaryotic promoter is stronger than the second eukaryotic promoter.

2. The expression vector of Claim 1, wherein the Coronavirus is pig, human or avian coronavirus.

3. The expression vector of Claim 1, wherein the Coronavirus pig TGEV coronavirus, human 229E coronavirus or human SARS virus.

4. The expression vector of Claim 1, wherein the envelope protein gene has the sequence as set forth in SEQ ID NO: 1.

5. The expression vector of Claim 1, wherein the membrane protein gene has the sequence as set forth in SEQ ID NO: 2.

6. The expression vector of Claim 1, wherein the SpikeCT gene has the sequence as set forth in SEQ ID NO: 3.

7. The expression vector of Claim 1, which further comprises a replication origin for the propagation plasmid in a host cell.

8. The expression vector of Claim 1, which further comprises an antibiotics-resistant gene as the selection marker.

9. The expression vector of Claim 1, wherein the first and second transcription units have polyA signal.

10. The expression vector of Claim 9, wherein the polyA signal BGH polyA signal.

11. The expression vector of Claim 1, which has the sequence as set forth in SEQID NO:49.

12. The expression vector of Claim 1, wherein the SpikeCT gene encodes C-terminal heptad repeat located upstream of an aromatic residue-rich region juxtaposed to the transmembrane segment of Spike protein of coronavirus.

13. The expression vector of Claim 1, the multiple cloning site comprises the restriction site selected from the group consisting of Smal, BsaB I, EcoR V and BspE I.

14. The expression vector of Claim 1, wherein the Class I viral fusion protein gene is selected from the group consisting of gp160 of HIV, HA of influenza virus and Spike of SARS virus.

15. The expression vector of Claim 1, wherein the first eukaryotic promoter is selected from the group consisting of CMV, SV40, RSV, HIV-1 LTR, hybrid Beta-actin/CMV promoter enhancer, muscle-specific desmin, creatine kinase, beta-Actin promoter, EF1 alpha, Ubiquitin promoter.

16. The expression vector of Claim 1, wherein the first eukaryotic promoter is selected from the group consisting of pCMV, Hybrid Beta-actin/CMV promoter enhancer and beta-Actin promoter.

17. The expression vector of Claim 1, wherein the second eukaryotic promoter is selected from the group consisting of CMV, SV40, RSV, HIV-1 LTR, hybrid Beta-actin/CMV promoter enhancer, muscle-specific desmin, creatine kinase, beta-Actin promoter, EF1alpha, Ubiquitin promoter.

18. The expression vector of Claim 1, wherein the second eukaryotic promoter is selected from the group consisting of pCMV, SV40, beta-Actin and EF1 alpha promoter.


SEQUENCE LISTING

<110>  DNASHUTTLE Biopharm Co., Ltd.

<120>  AN EXPRESSION VECTOR ENCODING CORONAVIRUS-LIKE PARTICLE

<130>  NONE

<140>  US 10/855,490
<141>  2004-05-28

<160>  49

<170>  PatentIn version 3.3

<210>  1
<211>  234
<212>  DNA
<213>  SARS E gene

<400>  1
atggcatact cttttgtgtc tgaggaaact ggcactctga tcgtgaactc tgtactgctg      60

tttctcgctt ttgtggtatt cctgctggtc actctcgcta tcctcactgc tcttcgtctg     120

tgtgcctact gttgtaatat cgtgaacgtg tctctggtta agcctactgt gtatgtgtat     180

tctcgtgtga aaaatctcaa ttcttctgaa ggagttcccg atctgctggt ctag           234


<210>  2
<211>  666
<212>  DNA
<213>  SARS M gene

<400>  2
atggcagata acggcactat tactgtggag gaactgaaac aactgctgga acaatggaac      60

ctcgtaatcg gctttctctt tctggcttgg attatgttgt tacagtttgc gtattctaat     120

cgtaaccgtt tcctctacat tattaagctc gttttcctgt ggttgttgtg gcctgtaact     180

cttgcttgct ttgtgcttgc tgctgtctat cgtatcaact gggttactgg tggtattgct     240

atcgctatgg cttgtattgt aggcttgatg tggctgtctt atttcgttgc ttctttccgt     300

ctgtttgctc gtactcgctc tatgtggtcc tttaatcctg agactaatat cctgctgaat     360

gttccgctcc gtggtactat cgttactaga ccgctgatgg aatctgaact ggttattggt     420

gccgtcatta tccgtggtca tttgcgtatg gctggtcact ctctgggtcg ttgcgatatt     480

aaggatctgc caaaggaaat cactgtagcc acttctcgta ctctgtctta ctataaactc     540

ggtgcatcgc aacgtgtggg aactgattcg ggcttcgctg cgtataatcg ttatcgtatt     600

ggcaactata aactgaacac cgaccacgca ggctctaatg acaacatcgc tctcctcgtt     660

cagtga                                                                666


<210>  3
<211>  327
<212>  DNA
<213>  SpikeCT gene

<400>  3
gatatctccg gaatcaatgc gagcgtagtg aacatccaga aagagattga ccgtttgaat      60

gaagttgcta aaaatctgaa tgaacctctg attgacctcc aagaactcgg caaatatgag     120

caatacatta aatggccttg gtatgtctgg ttgggtttca tcgcaggtct catcgctatc 180

gttatggtga ctattctgct gtgttgtatg acttcttgtt gctcttgtct gaaaggtgcg 240

tgttcttgtg gttcttgttg taagtttgat gaggatgatt ctgagccagt tctgaagggt 300

gtgaagctgc attataccta gttcgaa 327

<210> 4
<211> 34
<212> DNA
<213> E1L primer

<400> 4
accatggcat actcttttgt gtctgaggaa actg 34

<210> 5
<211> 51
<212> DNA
<213> E2L primer

<400> 5
acagcagtac agagttcacg atcagagtgc cagtttcctc agacacaaaa g 51

<210> 6
<211> 51
<212> DNA
<213> E3L primer

<400> 6
tgaccagcag gaataccaca aaagcgagaa acagcagtac agagttcacg a 51

<210> 7
<211> 54
<212> DNA
<213> E4L primer

<400> 7
gcacacagac gaagagcagt gaggatagcg agagtgacca gcaggaatac caca 54

<210> 8
<211> 52
<212> DNA
<213> E5L primer

<400> 8
accagagaca cgttcacgat attacaacag taggcacaca gacgaagagc ag 52

<210> 9
<211> 58
<212> DNA
<213> E6L primer

<400> 9
gatttttcac acgagaatac acatacacag taggcttaac cagagacacg ttcacgat 58

<210> 10
<211> 62
<212> DNA
<213> E7L primer

<400> 10

tctagaccag cagatcggga actccttcag aagaattgag atttttcaca cgagaataca 60

ca 62

<210> 11
<211> 20
<212> DNA
<213> E8L primer

<400> 11
tctagaccag cagatcggga 20

<210> 12
<211> 22
<212> DNA
<213> M1-1U primer

<400> 12
tgatcatggc agataacggc ac 22

<210> 13
<211> 37
<212> DNA
<213> M1U primer

<400> 13
tgatcatggc agataacggc actattactg tggagga 37

<210> 14
<211> 49
<212> DNA
<213> M1L primer

<400> 14
gttccattgt tccagcagtt gtttcagttc ctccacagta atagtgccg 49

<210> 15
<211> 49
<212> DNA
<213> M2L primer

<400> 15
aagccagaaa gagaaagccg attacgaggt tccattgttc cagcagttg 49

<210> 16
<211> 50
<212> DNA
<213> M3L primer

<400> 16
agaatacgca aactgtaaca acataatcca agccagaaag agaaagccga 50

<210> 17
<211> 57
<212> DNA
<213> M4L primer

<400> 17
cgagcttaat aatgtagagg aaacggttac gattagaata cgcaaactgt aacaaca 57

<210> 18
<211> 56

<212>  DNA
<213>  M5L primer

<400>  18
caagagttac aggccacaac aaccacagga aaacgagctt aataatgtag aggaaa          56


<210>  19
<211>  55
<212>  DNA
<213>  M6L primer

<400>  19
ttgatacgat agacagcagc aagcacaaag caagcaagag ttacaggcca caaca          55


<210>  20
<211>  58
<212>  DNA
<213>  M7L primer

<400>  20
caagccatag cgatagcaat accaccagta acccagttga tacgatagac agcagcaa          58


<210>  21
<211>  56
<212>  DNA
<213>  M8L primer

<400>  21
aacgaaataa gacagccaca tcaagcctac aatacaagcc atagcgatag caatac          56


<210>  22
<211>  59
<212>  DNA
<213>  M9L primer

<400>  22
acatagagcg agtacgagca aacagacgga aagaagcaac gaaataagac agccacatc          59


<210>  23
<211>  20
<212>  DNA
<213>  M10U primer

<400>  23
tcctgctgaa tgttccgctc                                                  20


<210>  24
<211>  65
<212>  DNA
<213>  M10L primer

<400>  24
gagcggaaca ttcagcagga tattagtctc aggattaaag gaccacatag agcgagtacg          60

agcaa                                                                  65


<210>  25
<211>  50
<212>  DNA
<213>  M11L primer

<400>  25
catcagcggt ctagtaacga tagtaccacg gagcggaaca ttcagcagga                  50

<210>  26
<211>  60
<212>  DNA
<213>  M12L primer

<400>  26
accacggata atgacggcac caataaccag ttcagattcc atcagcggtc tagtaacgat     60


<210>  27
<211>  31
<212>  DNA
<213>  M13L primer

<400>  27
atacgcaaat gaccacggat aatgacggca c                                    31


<210>  28
<211>  45
<212>  DNA
<213>  M14L primer

<400>  28
aacgacccag agagtgacca gccatacgca aatgaccacg gataa                     45


<210>  29
<211>  51
<212>  DNA
<213>  M15L primer

<400>  29
tacagtgatt tcctttggca gatccttaat atcgcaacga cccagagagt g              51


<210>  30
<211>  57
<212>  DNA
<213>  M16L primer

<400>  30
ccgagtttat agtaagacag agtacgagaa gtggctacag tgatttcctt tggcaga        57


<210>  31
<211>  58
<212>  DNA
<213>  M17L primer

<400>  31
cgaagcccga atcagttccc acacgttgcg atgcaccgag tttatagtaa gacagagt       58


<210>  32
<211>  58
<212>  DNA
<213>  M18L primer

<400>  32
cagtttatag ttgccaatac gataacgatt atacgcagcg aagcccgaat cagttccc       58


<210>  33
<211>  62
<212>  DNA
<213>  M19L primer

<400> 33
gagagcgatg ttgtcattag agcctgcgtg gtcggtgttc agtttatagt tgccaatacg 60

at 62

<210> 34
<211> 34
<212> DNA
<213> M20L primer

<400> 34
tcactgaacg aggagagcga tgttgtcatt agag 34

<210> 35
<211> 26
<212> DNA
<213> SpikeCTup-EcoRV primer

<400> 35
gatatctccg gaatcaatgc gagcgt 26

<210> 36
<211> 35
<212> DNA
<213> DI-1U/BspE I primer

<400> 36
tccggaatca atgcgagcgt agtgaacatc cagaa 35

<210> 37
<211> 48
<212> DNA
<213> DI-1L primer

<400> 37
gcaacttcat tcaaacggtc aatctctttc tggatgttca ctacgctc 48

<210> 38
<211> 45
<212> DNA
<213> DI-2L primer

<400> 38
atcagagatt cattcagatt tttagcaact tcattcaaac ggtca 45

<210> 39
<211> 51
<212> DNA
<213> DI-3L primer

<400> 39
tcatatttgc cgagttcttg gaggtcaatc agagattcat tcagattttt a 51

<210> 40
<211> 43
<212> DNA
<213> DI-4L primer

<400> 40
caaggccatt taatgtattg ctcatatttg ccgagttctt gga 43

<210> 41

<211>  50
<212>  DNA
<213>  DI-5L primer

<400>  41
acctgcgatg aaacccaacc agacatacca aggccattta atgtattgct                50


<210>  42
<211>  50
<212>  DNA
<213>  DI-6L primer

<400>  42
cagaatagtc accataacga tagcgatgag acctgcgatg aaacccaacc                50


<210>  43
<211>  50
<212>  DNA
<213>  DI-7L primer

<400>  43
agagcaacaa gaagtcatac aacacagcag aatagtcacc ataacgatag                50


<210>  44
<211>  50
<212>  DNA
<213>  DI-8L primer

<400>  44
accacaagaa cacgcacctt tcagacaaga gcaacaagaa gtcatacaac                50


<210>  45
<211>  50
<212>  DNA
<213>  DI-9L primer

<400>  45
gaatcatcct catcaaactt acaacaagaa ccacaagaac acgcaccttt                50


<210>  46
<211>  49
<212>  DNA
<213>  DI-10L primer

<400>  46
gcttcacacc cttcagaact ggctcagaat catcctcatc aaacttaca                 49


<210>  47
<211>  35
<212>  DNA
<213>  DI-11L primer

<400>  47
cctaggtata atgcagcttc acacccttca gaact                                35


<210>  48
<211>  27
<212>  DNA
<213>  SpikeCTdn-BstB I primer

<400>  48
ttcgaactag gtataatgca gcttcac                                         27

<210> 49
<211> 4800
<212> DNA
<213> CoVLP cloning vector

<400> 49
gacggatcgg gagatcttca atattggcca ttagccatat tattcattgg ttatatagca 60

taaatcaata ttggctattg gccattgcat acgttgtatc tatatcataa tatgtacatt 120

tatattggct catgtccaat atgaccgcca tgttggcatt gattattgac tagttattaa 180

tagtaatcaa ttacggggtc attagttcat agcccatata tggagttccg cgttacataa 240

cttacggtaa atggcccgcc tggctgaccg cccaacgacc cccgcccatt gacgtcaata 300

atgacgtatg ttcccatagt aacgccaata gggactttcc attgacgtca atgggtggag 360

tatttacggt aaactgccca cttggcagta catcaagtgt atcatatgcc aagtccgccc 420

cctattgacg tcaatgacgg taaatggccc gcctggcatt atgcccagta catgacctta 480

cgggactttc ctacttggca gtacatctac gtattagtca tcgctattac catggtgatg 540

cggttttggc agtacaccaa tgggcgtgga tagcggtttg actcacgggg atttccaagt 600

ctccacccca ttgacgtcaa tgggagtttg ttttggcacc aaaatcaacg ggactttcca 660

aaatgtcgta acaactgcga tcgcccgccc cgttgacgca aatgggcggt aggcgtgtac 720

ggtgggaggt ctatataagc agagctcgtt tagtgaaccg tcagatcact agaagcttta 780

ttgcggtagt ttatcacagt taaattgcta acgcagtcag tgcttctgac acaacagtct 840

cgaacttaag ctgcagtgac tctcttaagg tagccttgca gaagttggtc gtgaggcact 900

gggcaggtaa gtatcaaggt tacaagacag gtttaaggag accaatagaa actgggcttg 960

tcgagacaga gaagactctt gcgtttctga taggcaccta ttggtcttac tgacatccac 1020

tttgcctttc tctccacagg tgtccactcc cagttcaatt acagctctta aggctagagt 1080

acttaatacg actcactata ggctagcgct accggactca gatcatggca gataacggca 1140

ctattactgt ggaggaactg aaacaactgc tggaacaatg gaacctcgta atcggctttc 1200

tctttctggc ttggattatg ttgttacagt ttgcgtattc taatcgtaac cgtttcctct 1260

acattattaa gctcgttttc ctgtggttgt tgtggcctgt aactcttgct tgctttgtgc 1320

ttgctgctgt ctatcgtatc aactgggtta ctggtggtat tgctatcgct atggcttgta 1380

ttgtaggctt gatgtggctg tcttatttcg ttgcttcttt ccgtctgttt gctcgtactc 1440

gctctatgtg gtcctttaat cctgagacta atatcctgct gaatgttccg ctccgtggta 1500

ctatcgttac tagaccgctg atggaatctg aactggttat tggtgccgtc attatccgtg 1560

gtcatttgcg tatggctggt cactctctgg gtcgttgcga tattaaggat ctgccaaagg 1620

aaatcactgt agccacttct cgtactctgt cttactataa actcggtgca tcgcaacgtg 1680

tgggaactga ttcgggcttc gctgcgtata atcgttatcg tattggcaac tataaactga 1740

acaccgacca cgcaggctct aatgacaaca tcgctctcct cgttcagtga aatcactagt 1800

gcggccgcag gtaccgagct cggatccgcc cctctccctc ccccccccct aacgttactg 1860

gccgaagccg cttggaataa ggccggtgtg cgtttgtcta tatgttattt tccaccatat 1920

tgccgtcttt tggcaatgtg agggcccgga aacctggccc tgtcttcttg acgagcattc 1980
ctaggggtct ttcccctctc gccaaaggaa tgcaaggtct gttgaatgtc gtgaaggaag 2040
cagttcctct ggaagcttct tgaagacaaa caacgtctgt agcgaccctt tgcaggcagc 2100
ggaacccccc acctggcgac aggtgcctct gcggccaaaa gccacgtgta taagatacac 2160
ctgcaaaggc ggcacaaccc cagtgccacg ttgtgagttg gatagttgtg gaaagagtca 2220
aatggctctc ctcaagcgta ttcaacaagg ggctgaagga tgcccagaag gtaccccatt 2280
gtatgggatc tgatctgggg cctcggtgca catgctttac atgtgtttag tcgaggttaa 2340
aaaaacgtct aggccccccg aaccacgggg acgtggtttt cctttgaaaa acacgatgat 2400
aatatggcca caaccatggc atactctttt gtgtctgagg aaactggcac tctgatcgtg 2460
aactctgtac tgctgtttct cgcttttgtg gtattcctgc tggtcactct cgctatcctc 2520
actgctcttc gtctgtgtgc ctactgttgt aatatcgtga acgtgtctct ggttaagcct 2580
actgtgtatg tgtattctcg tgtgaaaaat ctcaattctt ctgaaggagt tcccgatctg 2640
ctggtctaga gggcccgttt aaacccgctg atcagcctcg actgtgcctt ctagttgcca 2700
gccatctgtt gtttgcccct cccccgtgcc ttccttgacc ctggaaggtg ccactcccac 2760
tgtcctttcc taataaaatg aggaaattgc atcgcattgt ctgagtaggt gtcattctat 2820
tctggggggt ggggtggggc aggacagcaa gggggaggat tgggaagaca atagcaggca 2880
tgctggggat gcggtgggct ctatggcttc tgaggcggaa agaaccagct ggggctctag 2940
ggggtatccc cacgcgccct gtagcggcgc attaagcgcg gcgggtgtgg tggttacgcg 3000
cagcgtgacc gctacacttg ccagcgccct agcgcccgct cctttcgctt tcttcccttc 3060
ctttctcgcc acgttcgccg gctttccccg tcaagctcta aatcgggggc tccctttagg 3120
gttccgattt agtgctttac ggcacctcga ccccaaaaaa cttgattagg gtgatggttc 3180
acgtagtggg ccatcgccct gatagacggt ttttcgccct ttgacgttgg agtccacgtt 3240
ctttaatagt ggactcttgt tccaaactgg aacaacactc aaccctatct cggtctattc 3300
ttttgattta taagggattt tgccgatttc ggcctattgg ttaaaaaatg agctgattta 3360
acaaaaattt aacgcgaatt aattctgtgg aatgtgtgtc agttagggtg tggaaagtcc 3420
ccaggctccc cagcaggcag aagtatgcaa agcatgcatc tcaattagtc agcaaccagg 3480
tgtggaaagt ccccaggctc cccagcaggc agaagtatgc aaagcatgca tctcaattag 3540
tcagcaacca tagtcccgcc cctaactccg cccatcccgc ccctaactcc gcccagttcc 3600
gcccattctc cgccccatgg ctgactaatt ttttttattt atgcagaggc cgaggccgcc 3660
tctgcctctg agctattcca gaagtagtga ggaggctttt ttggaggcct aggcttttgc 3720
aaaaagctcc cgggagcttg tatatccatt ttcggatctg atcaagagac aggatgacta 3780
gtgattgata tctccggaat caatgcgagc gtagtgaaca tccagaaaga gattgaccgt 3840
ttgaatgaag ttgctaaaaa tctgaatgaa cctctgattg acctccaaga actcggcaaa 3900
tatgagcaat acattaaatg gccttggtat gtctggttgg gtttcatcgc aggtctcatc 3960
gctatcgtta tggtgactat tctgctgtgt tgtatgactt cttgttgctc ttgtctgaaa 4020

ggtgcgtgtt cttgtggttc ttgttgtaag tttgatgagg atgattctga gccagttctg 4080

aagggtgtga agctgcatta tacctagttc gaaatgaccg accaagcgac gcccaacctg 4140

ccatcacgag atttcgattc caccgccgcc ttctatgaaa ggttgggctt cggaatcgtt 4200

ttccgggacg ccggctggat gatcctccag cgcggggatc tcatgctgga gttcttcgcc 4260

caccccaact tgtttattgc agcttataat ggttacaaat aaagcaatag catcacaaat 4320

ttcacaaata aagcattttt ttcactgcat tctagttgtg gtttgtccaa actcatcaat 4380

gtatcttatc atgtctgtat accgtcgacc tctagctaga gcttggcgta atcatggtca 4440

tagctgtttc ctgtgtgaaa ttgttatccg ctcacaattc cacacaacat acgagccgga 4500

agcataaagt gtaaagcctg gggtgcctaa tgagtgagct aactcacatt aattgcgttg 4560

cgctcactgc ccgctttcca gtcgggaaac ctgtcgtgcc agctgcatta atgaatcggc 4620

caacgcgcgg ggagaggcgg tttgcgtatt gggcgctctt ccgcttcctc gctcactgac 4680

tcgctgcgct cggtcgttcg gctgcggcga gcggtatcag ctcactcaaa ggcggtaata 4740

cggttatcca cagaatcagg ggataacgca ggaaagaaca tgtgagcaaa aggccagcaa 4800